# EUROPEAN PATENT SPECIFICATION

(11) **EP 4 483 825 B1**
(45) Date of publication and mention of the grant of the patent: **14.01.2026**
(21) Application number: 24156480.6
(22) Date of filing: 08.02.2024
(51) Int. Cl.: A61B 17/80, A61B 17/86

(54) **SCREW WITH VARIABLE-ANGLE FIXATION INSERTABLE INTO A THREADED HOLE IN A BONE PLATE**
SCHRAUBE MIT WINKELVERSTELLBARER FIXIERUNG, DIE IN EIN GEWINDELOCH IN EINER KNOCHENPLATTE EINGESETZT WERDEN KANN
VIS À FIXATION À ANGLE VARIABLE INSÉRABLE DANS UN TROU FILETÉ D'UNE PLAQUE OSSEUSE

(30) Priority: 27.06.2023 PT 2023118757
(43) Date of publication of application: 01.01.2025
(73) Proprietor: ASTROLABE - FABRICAÇÃO DE IMPLANTES MÉDICOS, LDA, P-2660-360 São Julião do Tojal (PT)
(72) Inventor: Klain, Eduardo, 13504-112 São Paulo (BR)
(74) Representative: Moniz Pereira, Manuel

(56) References cited:
- EP-A2- 1 767 160
- US-A1- 2004 260 291
- US-A1- 2015 190 185
- US-A1- 2018 235 681

## Description

### Technical field of the invention

The present invention falls within the area of orthopaedic medical devices, more precisely a bone fixation screw with variable-angle fixation to be inserted into a threaded hole in a bone plate.

### Scope of the invention

Bone plates and screws are implants used to provide structural support to bone while promoting osteosynthesis and bone healing after fractures or osteotomies. Bone plates and screws allow stabilization, restriction of bone movement, fracture reduction, and support of bone fragments, while controlling the anatomical alignment of the segments subjected to intervention.

Bone plates are used to provide structural support for the fixation of several types of bone fractures. Bone plates are attached to the bone to "reduce" the fracture, i.e., to align and bring the bone fragments closer together to facilitate the natural growth of the bone and the healing of the body. Bone reduction is generally achieved using non-locking screws. Non-locking screws have threaded rods and a spherical head, being anchored through holes in the bone plate and in the several bone fragments, which, when tightened, pull the bone fragments together under a compressive load against the plate. However, due to the dynamic loading caused by physiological movement, non-locking screws can back out over time.

Differently, locking screws are screws that provide fixation to both the bone and the locking plate, as the head of the screw is threaded, which allows it to be anchored to a threaded hole in the plate.

Variable-angle screws are locking screws that can be inserted into the plate's threaded hole at different angles. Variable-angle screws allow angular variation of fixation and can be fixed to the plate defining a conical insertion vector. This conical vector is determined by the angular variation that the screw describes when inserted into the plate: when a screw is inserted perpendicular to the surface of the plate, and the longitudinal axis of the screw coincides with the central axis of the hole in the plate, the angle of insertion is 0 degrees, thus describing a conical vector of 0 degrees. A screw that is inserted into the plate hole and whose insertion angle corresponds to 6 degrees between the longitudinal axis of the screw and the central axis of the plate hole, will describe a total conical insertion vector of 12 degrees, since the 6 degrees of insertion can be defined over 360 degrees around the central axis of the hole. This type of screw was developed to avoid joint surfaces or regions of insufficient bone density (cancellous bone) and/or covering fragments that would not be accessible through the use of other conventional screws.

Variable-angle screws can be applied to different fracture patterns, allowing a greater angle range for reducing fracture fragments. The variable-angle function of the screws allows polyaxially coupling of the variable-angle screw to the plate, enabling the choice of the most favourable angle to guide the variable-angle screw while promoting an effective locking mechanism.

Variable-angle screws known in the state of the art achieve the variable-angle function by including grooves along the entire variable-angle screw head, which enable screwing of the variable-angle screw head into the plate hole. This solution presents the variable-angle function, but with the drawback that the anchoring of the screw head to the plate is less secure since the threaded connection between the screw and the plate is discontinuous along the total length of the screw head.

For this reason, the need for a more secure threaded connection between the variable-angle screw and the plate hole was identified.

### Background of the Invention

Several prior states of the art publications were found that refer to variable-angle screws. However, many of these publications refer to variable-angle screws with heads that have grooves along the entire height of the head.

Examples can be found in patent document KR20210062857A, which refers to a "Variable-Angle Bone Fixation Screw", or in patent document WO2017192853A1, which discloses "Variable-Angle Locking Screws and Bone Plate Systems Including Variable-Angle Locking Screws", or even patent document CN104665910A, which presents a "Small Bone Clamping Device", and which refers to a bone fixation device comprising a screw and a plate. The screw comprises a groove along the entire screw head, with the head geometry being defined by two cones.

Patent document EP3941373A1 describes plates for internal fixation of bone fractures with three different types of recesses for their screws.

Patent document US 2004/260291 A1 describes a bone screw with a shoulder region (may be frustoconical or non-tapered) and a tapping structure (e.g., flutes) for threading into bone plates. The shoulder region (neck) may taper toward the shank, but this is not part of the head's threaded geometry.

### Advantages of the invention

The solutions described previously and already known in the state of the art present a few limitations and differences compared to the invention now proposed.

Regarding patent document CN104665910A, there are a few fundamental differences compared to the present invention.

In the present invention, the variable-angle screw allows the continuous screwing of the thread fillets in contact with the plate, in the upper region of the screw head thread, which ensures better fixation of the screw to the plate.

The major technical advantage that the present invention presents in relation to the devices referred to in the previous chapter, is that the grooves, by being placed only in the lower region of the head instead of being placed along the entire length of the thread, allow the upper part of the head to have complete thread fillets, i.e., without discontinuities. Thus, when screwing the screw into the plate, regardless of the angle allowed by it, the upper fillets of the thread that screw into the plate will have a complete winding, ensuring that the contact between the plate thread and the screw is made more securely, since there is more material establishing that contact. The same does not happen with the devices mentioned above, because as the groove is made along the entire length of the thread, contact between the plate thread and the screw will only be established in areas where there is no groove. Thus, the contact between threads is discontinuous and inevitably less secure than that presented by the screw of the present invention.

Additionally, due to the fact that it has a threaded head with a double frustoconical geometry, a less accentuated entry relief is established on the plate: while the threaded spherical head, in the lower part of the plate, quickly decreases in size until it reaches the body of the screw, the double frustoconical geometry has a less accentuated decrease in size from the top of the head until it reaches the screw body, which allows better tracking of the screw head in relation to the plate hole. In other words, there is the advantage that the head screw with double frustoconical geometry allows for a greater volume of material involved in screwing the screw into the plate hole, once again ensuring greater security in the connection between plate and screw.

### Brief description of the drawings

These and other features can be easily understood in the attached drawings, which should be considered mere examples and in no way restrict the scope of the invention. In the drawings, and for illustrative purposes, the measurements of some of the constituent parts may be exaggerated and not drawn to scale. The absolute dimensions and the relative dimensions do not correspond to the actual ratios for the invention.

In a preferred embodiment:

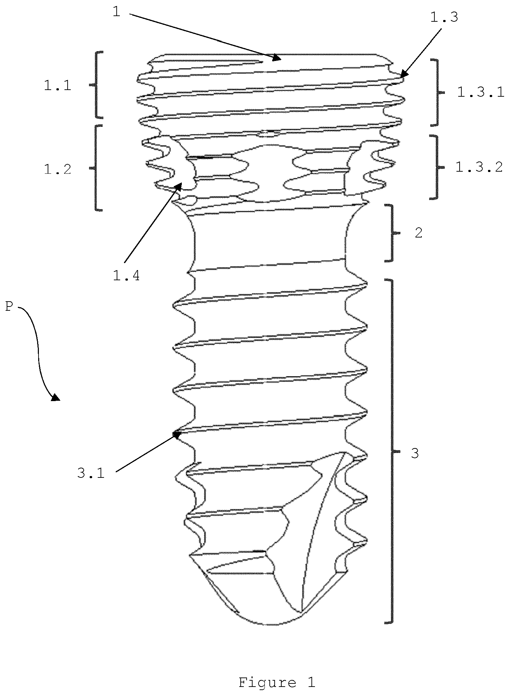

In Figure 1 it is possible to observe a front perspective view of the screw of the invention, with an indication of the screw's constituent parts.

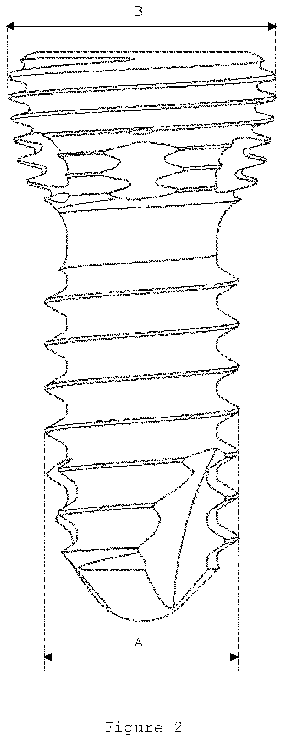

In Figure 2 it is possible to observe a front perspective view of the screw of the invention, with an indication of where the measurements of the diameters of the rod and head are taken.

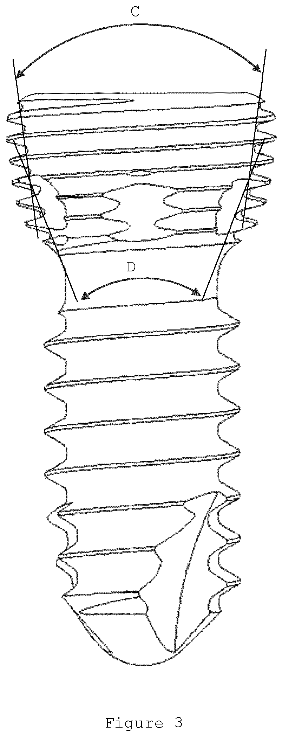

In Figure 3 it is possible to observe a front perspective view of the screw of the invention, with an indication of how the proximal and distal angles are measured.

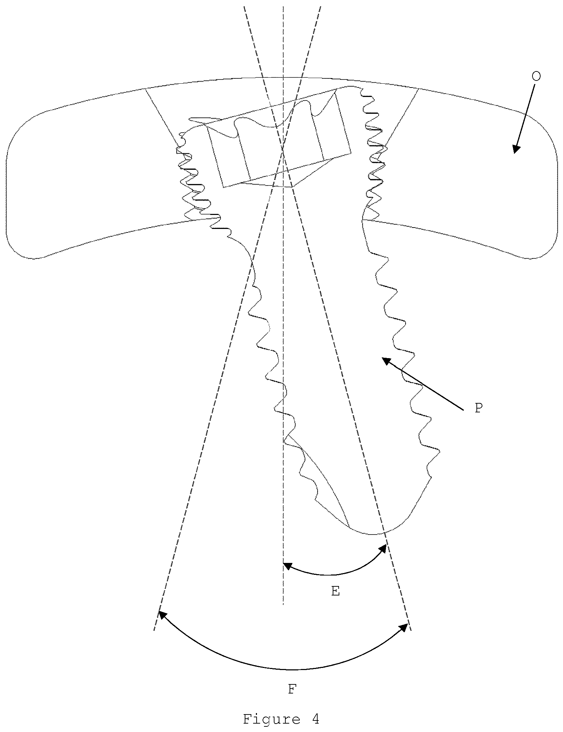

In Figure 4 it is possible to observe a front cutaway view of the screw of the invention and of the bone plate, with an indication of how the insertion angle and the conical insertion vector are measured.
In Figure 5 it is possible to observe a front cutaway view of the screw of the invention and of the bone plate, with an indication of where the gap and distance are measured.
In Figure 6 it is possible to observe a top perspective view of the screw of the invention.

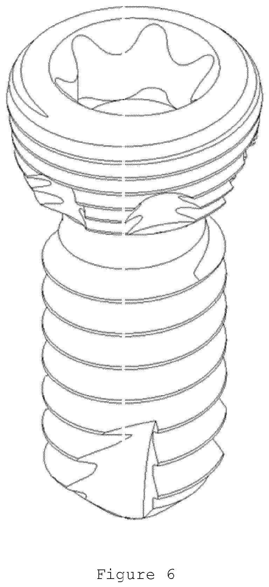

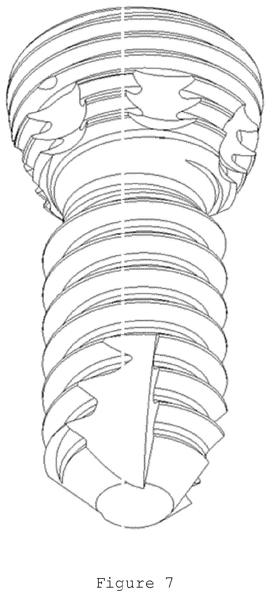

In Figure 6 it is possible to observe a lower perspective view of the screw of the invention.

- P.: screw
1. head
1.1 proximal portion
1.2 distal portion
1.3 head thread
1.3.1 upper region
1.3.2 lower region
1.4 grooves
2. neck
3. rod
3.1 thread of the rod
- A -: rod diameter
- B -: head diameter
- C -: proximal angle
- D -: distal angle
- E -: insertion angle
- F -: insertion conical vector
- G -: gap
- H -: distance
- O.: Bone plate

### Detailed description of the invention

The term "substantially" is understood to mean that the description of the shape or position of an element of the present invention is not mathematically or geometrically exact, but that the shape or position of an element of the present invention is recognized by a person skilled in the art as having generically or approximately the described shape or position.

As will be clear to a person skilled in the art, the application of the principles described here is not limited to the presented embodiments. Possible changes that may occur in the present invention, defined in number, remain within the scope of the present invention.

Additionally, although some embodiments present multiple new features, and as can be seen throughout this document, all features can be independent, and it is not essential that all be used in an embodiment.

Referring to the figures, the invention concerns a variable-angle bone fixation screw for insertion into a threaded hole in a bone plate (O).

The present invention is defined in claim 1 while preferred embodiments are set forth in the dependent claims.

The screw (P) of the invention includes a head (1) that has a proximal portion (1.1) and a distal portion (1.2) that define frustoconical geometries. The head (1) is threaded along its entire length and integrates a head thread (1.3), which has an upper region (1.3.1) and a lower region (1.3.2). The lower region (1.3.2) has grooves (1.4). The screw further includes a neck (2) extending from the lower end of the distal portion (1.2) of the head (1) and a threaded rod (3) extending from the lower end of the neck (2) and which includes a thread of the rod (3.1).

The bone fixation screw is configured to be coupled to the threaded hole of a bone plate (O).

The head (1) has a head diameter (B) that ranges from 0,003 m to 0,009 m. The rod diameter (A) ranges from 0,0024 m to 0,005 m.

The dimensions of the rod diameter (A) and of the head diameter (B) are dependent. This dependence is known from the state of the art, and below is a table with a few embodiments, which highlights the dimensions of the head diameter (B) as a function of the rod diameter (A):

| Rod diameter (A) | Head diameter (B) |
|---|---|
| 0,0024 m | 0,003 m to 0,006 m |
| 0,0027 m | 0,003 m to 0,006 m |
| 0,0035 m | 0,004 m to 0,007 m |
| 0,004 m | 0,005 m to 0,008 m |
| 0,005 m | 0,005 m to 0,009 m |

Additionally, the depth of the fillet of the head thread (1.3) of the screw (P) ranges from 0,0001 m to 0,0015 m.

The proximal portion (1.1) and the distal portion (1.2) have frustoconical geometries, which define the proximal angle (C) and the distal angle (D), respectively. The proximal angle (C), defined by the proximal portion (1.1) of the head ranges from 1° to 60°, more specifically from 5° to 30°, while the distal angle (D) defined by the distal portion (1.2) of the head ranges from 15° to 90°, more specifically from 30° to 60°, with the proximal angle (C) being smaller than the distal angle (D). These conicity values of the frustoconical geometries result in a tapering of the distal portion (1.2) in relation to the proximal portion (1.1) of the head (1), which will assist in the inclination of the screw as it is threaded into the bone plate hole (O).

Additionally, the screw is coupled so that it can be placed at an angle, i.e., so as to make an insertion angle (E) in relation to the central axis of the fixing hole that can range from 0° to 30°, more specifically from 0° to 15°, more specifically from 0° to 10°, thus creating a conical insertion vector (F) that can range from 0° to 60°, more specifically from 0° to 30°, more specifically from 0° to 20°.

The upper region (1.3.1) of the head thread (1.3) has thread segments that continuously fit into the threaded hole of the bone plate (O), while the lower region (1.3.2) of the head thread (1.3) has thread segments that fit discontinuously into the threaded hole.

The upper region (1.3.1) of the head thread (1.3) has a continuous thread, while the lower region (1.3.2) of the head thread (1.3) has a discontinuous thread. The discontinuity of the lower region (1.3.2) is defined as having at least one groove (1.4). Thus, the fact that the upper region (1.3.1) is continuous, allows a continuous fit into the threaded hole of the bone plate (O) in the referred region, enabling a secure fixation between the screw and the bone plate (O).

The discontinuities in the lower region (1.3.2) of the head thread (1.3) are achieved by the existence of at least one groove (1.4), in a preferred embodiment, six grooves (1.4), placed in a substantially equidistant position along the perimeter of the head (1) of the screw. Said grooves (1.4) have a substantially spherical shape with a radius that ranges from 0,0005 m to 0,0015 m, more specifically from 0,00075 m to 0,00125 m.

The existence of discontinuities in the lower region (1.3.2) of the head thread (1.3), together with the tapering of the distal portion (1.2) of the head (1), create a gap (G) between the screw and the hole, which enables the screw to be angled as it is inserted into the threaded hole in the bone plate (O).

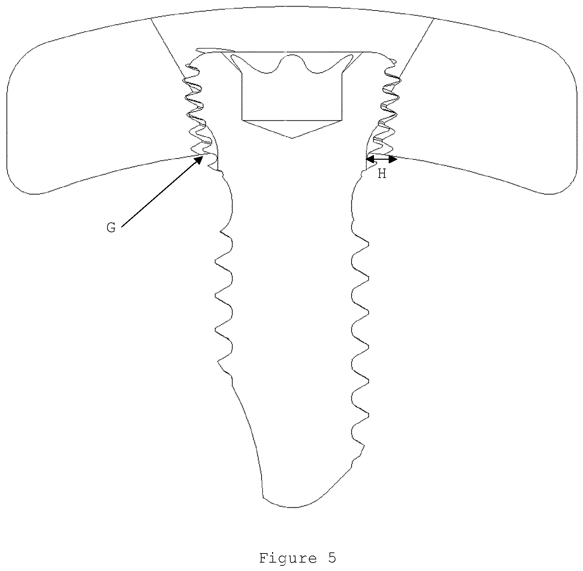

The existence of the gap (G) is evident in Figure 5, where it is possible to observe the screw when inserted into the bone plate (O) hole, with an insertion angle (E) of 0°. The distal portion (1.2), together with the grooves (1.4) of the screw, define a distance (H) between the distal limit of the bone plate (O) hole and the screw material, thus creating the gap (G).

The gap (G), when the screw is inserted with an insertion angle (E) greater than 0°, will enable the distance (H) to be such that it allows the screw to be inserted and screwed into the bone plate (O) plate hole without there being conflict between the screw material and the bone plate (O) hole.

When the insertion angle (E) is equal to 0°, the distance (H) is maximum, as is the gap (G). When the screw is inserted with the maximum insertion angle (E), the distance (H) is minimum, as is the gap (G).

Thus, the upper region (1.3.1) of the head thread (1.3) of the screw allows continuous coupling with the threaded hole of the bone plate (O), while the lower region (1.3.2) of the head thread (1.3), along with the distal portion (1.2), allow the variable-angle characteristic of the screw.

The combination of the double frustoconical geometry with the grooves (1.4) located in the lower region (1.3.2) of the head thread (1.3) allows obtaining threading between the head (1) of the screw and the hole in the bone plate (O) that makes up the conical insertion vector (F).

Below is a table with a few embodiments, highlighting the insertion angle (E) and consequently the conical insertion vector (F) according to the size of the rod diameter (A):

| Rod diameter (A) | Insertion angle (E) | Conical insertion vector (F) |
|---|---|---|
| 0,0024 m | 15° | 30° |
| 0,0027 m | 15° | 30° |
| 0,0035 m | 15° | 30° |
| 0,004 m | 15° | 30° |
| 0,005 m | 10° | 20° |

## Claims

1. A screw with variable-angle fixation insertable into a threaded hole of a bone plate (O), comprising:
- a head (1) threaded along its entire length, with a proximal portion (1.1) and a distal portion (1.2), that includes a head thread (1.3), which has an upper region (1.3.1) and a lower region (1.3.2);
- a neck (2) extending from the lower end of the distal portion (1.2) of the head (1) and a threaded rod (3) extending from the lower end of the neck (2) and which includes a thread of the rod (3.1);
- the lower region (1.3.2) of the head thread (1.3) has a discontinuous thread defined by comprising at least one groove (1.4);
**characterized in that** the head thread (1.3) has a double frustoconical geometry.

2. The screw according to the previous claim, **characterized in that** the proximal portion (1.1) and the distal portion (1.2) present frustoconical geometries.

3. The screw according to any of the previous claims, **characterized in that** the lower region (1.3.2) of the head thread (1.3) includes six grooves (1.4).

4. The screw according to any of the previous claims, **characterized in that** the grooves (1.4) are placed in a substantially equidistant position along the perimeter of the head (1) of the screw (P).

5. The screw according to any of the previous claims, **characterized in that** the grooves (1.4) have a substantially spherical shape with a radius that ranges from 0,0005 m to 0,0015 m.

6. The screw according to any of the previous claims, **characterized in that** the head (1) has a head diameter (B) ranging from 0,003 m to 0,009 m.

7. The screw according to any of the previous claims, **characterized in that** the rod diameter (A) ranges from 0,0024 m to 0,005 m.

8. The screw according to any of the previous claims, **characterized in that** the depth of the fillet of the head thread (1.3) of the screw (P) ranges from 0,0001 m to 0,0015 m.

9. The screw according to any of the claims 2 to 8, **characterized in that** the frustoconical geometry of the proximal portion (1.1) defines a proximal angle (C) and the frustoconical geometry of the distal portion (1.2) defines a distal angle (D).

10. The screw according to the previous claim, **characterized in that** the proximal angle (C) ranges from 1° to 60°.

11. The screw according to any of claims 9 to 10, **characterized in that** the distal angle (D) ranges from 15° to 90°.

12. The screw according to any of claims 9 to 11, **characterized in that** the proximal angle (C) is smaller than the distal angle (D).

## Patentansprüche

1. Schraube mit winkelstabiler Fixierung, einführbar in ein Gewindeloch einer Knochenplatte (O), umfassend:
- einen über seine gesamte Länge mit Gewinde versehenen Kopf (1) mit einem proximalen Abschnitt (1.1) und einem distalen Abschnitt (1.2), der ein Kopfgewinde (1.3) mit einem oberen Bereich (1.3.1) und einen unteren Bereich (1.3.2) aufweist;
- einen Hals (2), der sich vom unteren Ende des distalen Abschnitts (1.2) des Kopfes (1) erstreckt, und einen Gewindeschaft (3), der sich vom unteren Ende des Halses (2) erstreckt und ein Schaftgewinde (3.1) aufweist;
- wobei der untere Bereich (1.3.2) des Kopfgewindes (1.3) ein unterbrochenes Gewinde aufweist, dadurch definiert, dass es mindestens eine Nut (1.4) umfasst;
**dadurch gekennzeichnet, dass** das Kopfgewinde (1.3) eine doppelkegelstumpfförmige Geometrie aufweist.

2. Schraube nach dem vorhergehenden Anspruch, **dadurch gekennzeichnet, dass** der proximale Abschnitt (1.1) und der distale Abschnitt (1.2) kegelstumpfförmige Geometrien aufweisen.

3. Schraube nach einem der vorhergehenden Ansprüche, **dadurch gekennzeichnet, dass** der untere Bereich (1.3.2) des Kopfgewindes (1.3) sechs Nuten (1.4) aufweist.

4. Schraube nach einem der vorhergehenden Ansprüche, **dadurch gekennzeichnet, dass** die Nuten (1.4) in einer im Wesentlichen äquidistanten Position entlang des Umfangs des Kopfes (1) der Schraube (P) angeordnet sind.

5. Schraube nach einem der vorhergehenden Ansprüche, **dadurch gekennzeichnet, dass** die Nuten (1.4) eine im Wesentlichen sphärische Form mit einem Radius aufweisen, der von 0,0005 m bis 0,0015 m reicht.

6. Schraube nach einem der vorhergehenden Ansprüche, **dadurch gekennzeichnet, dass** der Kopf (1) einen Kopfdurchmesser (B) aufweist, der von 0,003 m bis 0,009 m reicht.

7. Schraube nach einem der vorhergehenden Ansprüche, **dadurch gekennzeichnet, dass** der Schaftdurchmesser (A) von 0,0024 m bis 0,005 m reicht.

8. Schraube nach einem der vorhergehenden Ansprüche, **dadurch gekennzeichnet, dass** die Tiefe der Gewinderundung des Kopfgewindes (1.3) der Schraube (P) von 0,0001 m bis 0,0015 reicht.

9. Schraube nach einem der Ansprüche 2 bis 8, **dadurch gekennzeichnet, dass** die kegelstumpfförmige Geometrie des proximalen Abschnitts (1.1) einen proximalen Winkel (C) und die kegelstumpfförmige Geometrie des distalen Abschnitts (1.2) einen distalen Winkel (D) definiert.

10. Schraube nach dem vorhergehenden Anspruch, **dadurch gekennzeichnet, dass** der proximale Winkel (C) von 1° bis 60° reicht.

11. Schraube nach einem der Ansprüche 9 bis 10, **dadurch gekennzeichnet, dass** der distale Winkel (D) von 15° bis 90° reicht.

12. Schraube nach einem der Ansprüche 9 bis 11, **dadurch gekennzeichnet, dass** der proximale Winkel (C) kleiner ist als der distale Winkel (D).

## Revendications

1. Une vis avec une fixation à angle variable, qui peut être insérée dans un trou fileté d'une plaque osseuse (O), comprenant:
- une tête (1) filetée sur toute sa longueur, avec une partie proximale (1.1) et une partie distale (1.2), qui est composée d'un filet de tête (1.3) qui a une zone supérieure (1.3.1) et une zone inférieure (1.3.2);
- un col (2) s'étendant à partir de l'extrémité inférieure de la partie distale (1.2) de la tête (1) et une tige filetée (3) s'étendant à partir de l'extrémité inférieure du col (2) et qui est composée d'un filetage de la tige (3.1);
- la zone inférieure (1.3.2) du filet de tête (1.3) dispose d'un filetage discontinu délimité par au moins une rainure (1.4);
**caractérisée en ce que** le filet de tête (1.3) a d'une géométrie tronconique double.

2. La vis selon la revendication précédente, **caractérisée en ce que** la partie proximale (1.1) et la partie distale (1.2) présentent des géométries tronconiques.

3. La vis selon l'une des revendications précédentes, **caractérisée en ce que** la zone inférieure (1.3.2) du filet de tête (1.3) est composée de six rainures (1.4).

4. La vis selon l'une des revendications précédentes, **caractérisée en ce que** les rainures (1.4) sont placées dans une position sensiblement équidistante le long du périmètre de la tête (1) de la vis (P).

5. La vis selon l'une des revendications précédentes, **caractérisée en ce que** les rainures (1.4) ont une forme sensiblement sphérique avec un rayon qui varie de 0,0005 m à 0,0015 m.

6. La vis selon l'une des revendications précédentes, **caractérisée en ce que** la tête (1) a un diamètre de tête (B) qui varie de 0,003 m à 0,009 m.

7. La vis selon l'une des revendications précédentes, **caractérisée en ce que** le diamètre de la tige (A) varie de 0,0024 m à 0,005 m.

8. La vis selon l'une des revendications précédentes, **caractérisée en ce que** la profondeur du filet du filet de tête (1.3) de la vis (P) varie de 0,0001 m à 0,0015 m.

9. La vis selon l'une des revendications 2 à 8, **caractérisée en ce que** la géométrie tronconique de la partie proximale (1.1) définit un angle proximal (C) et la géométrie tronconique de la partie distale (1.2) définit un angle distal (D).

10. La vis selon la revendication précédente, **caractérisée en ce que** l'angle proximal (C) varie de 1° à 60°.

11. La vis selon l'une des revendications 9 à 10, **caractérisée en ce que** l'angle distal (D) varie de 15° à 90°.

12. La vis selon l'une des revendications 9 à 11, **caractérisée en ce que** l'angle proximal (C) est inférieur à l'angle distal (D) .
